# EUROPÄISCHE PATENTANMELDUNG

(11) **EP 4 738 375 A1**
(43) Veröffentlichungstag der Anmeldung: **06.05.2026**
(21) Anmeldenummer: 25211538.1
(22) Anmeldetag: 28.10.2025
(51) Int. Cl.: G16H 20/10, G16H 80/00, A61B 5/00

(54) **VERFAHREN ZUR KLASSIFIKATION EINES PATIENTENZUSTANDES**

(30) Priorität: 29.10.2024 DE 102024131532
(71) Anmelder: MicuraPharm GmbH, 55270 Klein-Winternheim (DE)
(72) Erfinder: Mähringer-Kunz, Edgar, 55424 Münster-Sarmsheim (DE); Theuer, Heiko, 55129 Mainz (DE); Kettermann, Florian, 61118 Bad Vilbel (DE)
(74) Vertreter: Gottschald Patentanwälte Partnerschaft mbB

(57) **Zusammenfassung**

Die Erfindung betrifft ein Verfahren zur Klassifikation eines Patientenzustandes mittels einer Unterstützungsanordnung (1), wobei die Unterstützungsanordnung (1) eine Datenverarbeitungsanordnung (2), eine portable Medikamentenausgabeeinheit (3) und eine Sensoranordnung (4) aufweist, wobei die Datenverarbeitungsanordnung (2) eine Speicheranordnung (7) und eine Steuereinheit (8) aufweist, wobei die Steuereinheit (8) an der Medikamentenausgabeeinheit (3) angeordnet ist, wobei die Medikamentenausgabeeinheit (3) ein Lagersystem (9) mit mehreren Dosen mindestens eines Medikaments aufweist, wobei in der Speicheranordnung (7) ein Therapieplan mit Zeitpunkten (11) für die Einnahme der Dosen des Medikaments gespeichert ist, wobei die Steuereinheit (8) die Medikamentenausgabeeinheit (3) steuert, um die Dosen nach dem Therapieplan zu den Zeitpunkten (11) auszugeben, wobei die Sensoranordnung (4) mindestens einen Sensor (12) aufweist, der Gesundheitsdaten (13) des Patienten (P) in Zeitspannen nach den Zeitpunkten (11) erfasst. Es wird vorgeschlagen, dass mit dem Medikament assoziierte Wirkungsdaten über die Wirkung des Medikaments in den Zeitspannen in der Speicheranordnung (7) gespeichert sind, dass die Datenverarbeitungsanordnung (2) regelmäßig Eingangsdaten (16) miteinander korreliert und basierend auf der Korrelation einen jeweiligen Patientenzustand in eine aus mehreren Klassen einordnet, und, dass die Eingangsdaten (16) die Gesundheitsdaten (13) und die Wirkungsdaten umfassen.

## Beschreibung

Die vorliegende Erfindung betrifft ein Verfahren zur Klassifikation eines Patientenzustandes gemäß dem Oberbegriff von Anspruch 1, eine Verwendung einer Medikamentenausgabeeinheit in dem Verfahren gemäß Anspruch 13, einen Datenträger mit aggregierten und anonymisierten Daten gemäß Anspruch 14 sowie eine Unterstützungsanordnung zur Klassifikation eines Patientenzustandes gemäß dem Oberbegriff von Anspruch 15.

Patienten mit chronischen Erkrankungen müssen oft über längere Zeit ein oder mehrmals täglich Medikamente einnehmen. Gerade bei älteren und/oder multimorbiden Patienten kommt es vor, dass der Patient mehrere Medikamente in unterschiedlichen Rhythmen nach einem Therapieplan einnehmen muss. Oft wird die Behandlung umgesetzt, indem der Patient von einer Apotheke oder dgl. eine Medikamentenausgabeeinheit mit mehreren Fächern erhält, wobei jedes Fach einem Zeitpunkt zugeordnet ist und zum entsprechenden Zeitpunkt die im Fach enthaltenen Medikamente eingenommen werden sollen. Die Medikamentenausgabeeinheit kann beispielsweise eine Woche oder einen Monat abbilden und wird dann entsorgt (Einmalnutzung) oder wieder befüllt (Mehrfachnutzung).

Problematisch ist, dass die Compliance der Patienten, also die Einhaltung des Therapieplans, die einen wesentlichen Einfluss auf den Therapieerfolg hat, weder gut kontrollierbar noch im Durchschnitt besonders hoch ist. Selbst bei hoher Compliance ist die Einnahme mehrerer Medikamente für den Patienten oft herausfordernd, da die Wirkungen inklusive Nebenwirkungen und gegebenenfalls Wechselwirkungen der Medikamente gravierenden Einfluss auf das tägliche Leben haben können. Kommt dann noch ein schlechter Allgemeinzustand des Patienten und möglicherweise eine Pflegebedürftigkeit hinzu, wird es für einen behandelnden Arzt schwer, die Einflüsse der Medikamente abzuschätzen, auftretende Nebenwirkungen von echten Symptomen zu unterscheiden und wirksam gegenzusteuern.

Grundsätzlich sind Systeme wie Sturzsensoren und Hausnotrufe bekannt, um Patienten bei Stürzen und anderen Notfällen von Synkope bis Herzinfarkt schnell zu behandeln. Diese Systeme funktionieren jedoch unabhängig von der Medikation des Patienten.

Ein bekannter Stand der Technik (US 2023/0248614 A1) von dem die Erfindung ausgeht, betrifft ein Verfahren gemäß dem Oberbegriff von Anspruch 1. Bei dem bekannten Verfahren wird mittels einer Medikamentenausgabeeinheit die Einnahme der Medikamente und ein Gesundheitszustand des Patienten überwacht. Der Gesundheitszustand wird dadurch überwacht, dass ein Arzt o. dgl. einen akzeptablen Bereich für Gesundheitsparameter festlegt und ein Alarm ausgelöst wird, wenn dieser Bereich verlassen wird.

Somit ist ein Verfahren zur Klassifikation eines Patientenzustandes mittels einer Unterstützungsanordnung, wobei die Unterstützungsanordnung eine Datenverarbeitungsanordnung, eine portable Medikamentenausgabeeinheit und eine Sensoranordnung aufweist, wobei die Datenverarbeitungsanordnung eine Speicheranordnung und eine Steuereinheit aufweist, wobei die Steuereinheit an der Medikamentenausgabeeinheit angeordnet ist, wobei die Medikamentenausgabeeinheit ein Lagersystem mit mehreren Dosen mindestens eines Medikaments aufweist, wobei in der Speicheranordnung ein Therapieplan mit Zeitpunkten für die Einnahme der Dosen des Medikaments durch einen Patienten gespeichert ist, wobei die Steuereinheit die Medikamentenausgabeeinheit steuert, um die Dosen nach dem Therapieplan zu den Zeitpunkten an den Patienten auszugeben, wobei die Sensoranordnung mindestens einen Sensor aufweist, der Gesundheitsdaten des Patienten in Zeitspannen nach den Zeitpunkten erfasst, bekannt.

Es bleibt jedoch eine Herausforderung, eine ganzheitliche Therapie und Unterstützung eines Patienten technologisch zu unterstützen und weitere Einflussfaktoren auf das Wohlbefinden des Patienten zu identifizieren und insgesamt bei der Therapie und Unterstützung zu berücksichtigen.

Der Erfindung liegt das Problem zugrunde, das bekannte Verfahren derart auszugestalten und weiterzubilden, dass hinsichtlich der genannten Herausforderung eine weitere Optimierung erreicht wird.

Das obige Problem wird durch die Merkmale des kennzeichnenden Teils von Anspruch 1 gelöst.

Wesentlich ist die grundsätzliche Überlegung, dass Medikamentenwirkungen bei der Einschätzung eines Patientenzustandes berücksichtigt werden können, um die Einflüsse der Medikamente einerseits besser zu erforschen und andererseits bekannte Einflüsse von anderen Effekten, insbesondere Notfällen, zu trennen. Beispielsweise sind Pulssensoren wie Smartwatches inzwischen stark verbreitet. Grundsätzlich denkbar wäre daher, beim Überschreiten einer bestimmten Pulsfrequenz einen Alarm auszulösen. Das würde jedoch entweder zu einer geringen Sensitivität oder vielen Fehlauslösungen führen. Durch die Berücksichtigung von Medikamentenwirkungen, die erwartbar zu körperlichen Veränderungen führen, lässt sich eine verbesserte Detektion von verschiedenen Patientenzuständen realisieren.

Im Einzelnen wird vorgeschlagen, dass mit dem Medikament assoziierte Wirkungsdaten über die Wirkung des Medikaments in den Zeitspannen in der Speicheranordnung gespeichert sind, dass die Datenverarbeitungsanordnung regelmäßig Eingangsdaten miteinander korreliert und basierend auf der Korrelation einen jeweiligen Patientenzustand in eine aus mehreren Klassen einordnet, und, dass die Eingangsdaten die Gesundheitsdaten und die Wirkungsdaten umfassen.

Eine weitere Verbesserung ergibt sich, wenn zwischen mindestens einem regulären Patientenzustand und mehreren irregulären Patientenzuständen und zugehörigen Klassen unterschieden wird (Anspruch 2). Diverse irreguläre Patientenzustände (bspw. starke sportliche Aktivität oder begründeter Stress) sind kein Grund zur Besorgnis oder geben nur geringen Anlass zu einer Intervention. Die Genauigkeit der Detektion von problematischen irregulären Patientenzuständen lässt sich verbessern, wenn zwischen mehreren irregulären Zuständen unterschieden wird.

Einigen einem irregulären Patientenzustand zugeordneten Klassen kann eine Notreaktion zugeordnet sein (Anspruch 3). Denkbare Notreaktionen sind eine Alarmierung des Patienten selbst, eine Alarmierung eines Angehörigen oder Pflegedienstes und eine Alarmierung eines Notdienstes.

Bei der Einordnung des Patientenzustandes in die Klassen handelt es sich um ein Problem einer Mustererkennung in einer großen Menge an Daten, sodass die, gemäß Anspruch 4 vorgeschlagene, Verwendung von KI (trainiertes Maschinenlernmodell) vorteilhaft sein kann. Das trainierte Maschinenlernmodell kann darauf trainiert sein, Muster in den Eingangsdaten zu erkennen und Abweichungen von diesen Mustern zu erkennen. Solche Abweichungen können Anzeichen für Nebenwirkungen, Notfälle o.dgl. sein.

Weitere bevorzugte Eingangsdaten (Aktivitätsdaten und Stressdaten) sind Gegenstand der Ansprüche 5 und 6.

Patientenzustände lassen sich nicht vollständig klar abgegrenzt klassifizieren und können auch vom aktuellen Patientenzustand abhängen. Ein Notfall bei einem gerade sportlich aktiven Patienten erzeugt andere Gesundheitsdaten als ein Notfall bei einem schlafenden Patienten. Die Unterscheidung kann verbessert werden, wenn der aktuelle Patientenzustand bei der Einordnung berücksichtigt wird (Anspruch 7). So ergeben sich voneinander abhängige Klasseneinordnungen nach Art eines Zustandsautomaten (finite state machine).

Bei einer Ausgestaltung gemäß Anspruch 8 wird vorgeschlagen, dass für den Patienten aus den Eingangsdaten Referenzdaten ermittelt werden. Besonders vorteilhaft lassen sich diese Referenzdaten ermitteln, wenn festgestellt wurde, dass der Patient sich in einem regulären Patientenzustand befindet. So kann die Einordnung in die Klassen mit der Zeit verbessert werden und die Datenverarbeitungsanordnung lernt den Patienten kennen (Anspruch 9). Beispielsweise können so für Hochpulser andere Pulsfrequenz-Grenzwerte festgelegt werden als für andere Patienten. Das trainierte Maschinenlernmodell kann ebenfalls an den Patienten angepasst werden (Feintuning). Vorzugsweise ist vorgesehen, dass die Datenverarbeitungsanordnung das trainierte Maschinenlernmodell mit den Eingangsdaten und/oder den Referenzdaten nachtrainiert.

In einer Ausgestaltung gemäß Anspruch 10 wird vorgeschlagen, dass die Wirkung des Medikaments in den Gesundheitsdaten erkannt wird. Diese Wirkung kann aus den Gesundheitsdaten herausgerechnet werden oder anderweitig berücksichtigt werden, um die Einordnung in Klassen nachfolgend ohne Einfluss des Medikaments durchzuführen. Beispielsweise kann das trainierte Maschinenlernmodell darauf trainiert sein, Notfälle bei Patienten ohne Medikamenteneinfluss zu erkennen und der Medikamenteneinfluss kann herausgerechnet oder anderweitig berücksichtigt werden. Beispielsweise auf diese Art und Weise kann das Maschinenlernmodell trainiert werden, ohne Trainingsdaten von Patienten mit Medikamenteneinnahme zu nutzen.

In einer Ausgestaltung ist vorgesehen, dass die Speicheranordnung lokal beim Patienten, insbesondere an der Medikamentenausgabeeinheit, angeordnet ist. Die Daten können dann, insbesondere aus Datenschutzgründen und zu Zwecken der schnelleren und zuverlässigeren Bearbeitung, lokal gespeichert werden.

Ein weiterer Vorteil des vorschlagsgemäßen Verfahrens ist, dass die erhobenen Gesundheitsdaten einen direkten Bezug zu Medikamenten aufweisen. Werden die Eingangsdaten anonymisiert und aggregiert, lassen sich vielfältige Erkenntnisse zu Nebenwirkungen und Wechselwirkungen von Medikamenten gewinnen (Anspruch 11).

Anspruch 12 betrifft bevorzugte Ausgestaltungen der Medikamentenausgabeeinheit und der Möglichkeit, mehrere Medikamente vorzuhalten.

Nach einer weiteren Lehre gemäß Anspruch 13, der eigenständige Bedeutung zukommt, wird eine Verwendung einer Medikamentenausgabeeinheit in dem vorschlagsgemäßen Verfahren beansprucht.

Auf alle Ausführungen zu dem vorschlagsgemäßen Verfahren darf verwiesen werden.

Nach einer weiteren Lehre gemäß Anspruch 14, der ebenfalls eigenständige Bedeutung zukommt, wird ein Datenträger mit aggregierten und anonymisierten Daten erhalten durch das vorschlagsgemäße Verfahren beansprucht.

Auf alle Ausführungen zu dem vorschlagsgemäßen Verfahren und der vorschlagsgemäßen Verwendung darf verwiesen werden.

Nach einer weiteren Lehre gemäß Anspruch 15, der ebenfalls eigenständige Bedeutung zukommt, wird eine Unterstützungsanordnung zur Klassifikation eines Patientenzustandes beansprucht.

Dabei ist wesentlich, dass mit dem Medikament assoziierte Wirkungsdaten über die Wirkung des Medikaments in den Zeitspannen in der Speicheranordnung gespeichert sind, dass die Datenverarbeitungsanordnung regelmäßig Eingangsdaten miteinander korreliert und basierend auf der Korrelation einen jeweiligen Patientenzustand in eine aus mehreren Klassen einordnet, und, dass die Eingangsdaten die Gesundheitsdaten und die Wirkungsdaten umfassen.

Auf alle Ausführungen zu dem vorschlagsgemäßen Verfahren, der vorschlagsgemäßen Verwendung und dem vorschlagsgemäßen Datenträger darf verwiesen werden.

Im Folgenden wird die Erfindung anhand einer lediglich Ausführungsbeispiele darstellenden Zeichnung näher erläutert. In der Zeichnung zeigt
- Fig. 1: schematisch das Zusammenwirken der Bestandteile der Unterstützungsanordnung und
- Fig. 2: die Eingangsdaten, deren Verarbeitung und mögliche Notreaktionen.

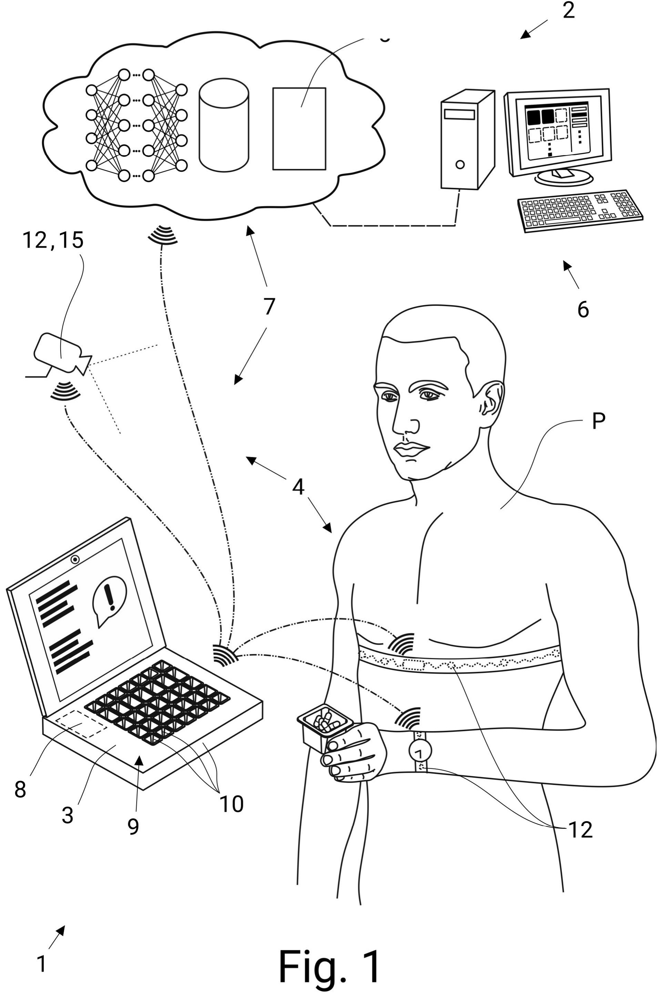

Vorgeschlagen wird ein Verfahren zur Klassifikation eines Patientenzustandes mittels einer Unterstützungsanordnung 1. Fig. 1 zeigt schematisch einen Patienten P und eine Unterstützungsanordnung 1, wobei die Unterstützungsanordnung 1 räumlich verteilt sein kann, wie durch die dargestellte Cloud angedeutet wird.

Die Unterstützungsanordnung 1 weist eine Datenverarbeitungsanordnung 2, eine portable Medikamentenausgabeeinheit 3 und eine Sensoranordnung 4 auf. Die Datenverarbeitungsanordnung 2 kann einen Cloud-Prozessor 5 und/oder ein Smartphone insbesondere mit einer dedizierten App und/oder einen lokalen Computer 6 mit dedizierter Software und/oder einem Webinterface zum Cloud-Prozessor 5 aufweisen.

Die Datenverarbeitungsanordnung 2 weist eine Speicheranordnung 7 und eine Steuereinheit 8 auf. Die Steuereinheit 8 ist an der Medikamentenausgabeeinheit 3 angeordnet. Denkbar ist, dass die Steuereinheit 8 den einzigen Prozessor der Datenverarbeitungsanordnung 2 aufweist, wie dargestellt kann die Datenverarbeitungsanordnung 2 jedoch auch mehrere verteilte Prozessoren aufweisen. Die Steuereinheit 8 kann einen lokalen Speicher aufweisen, der der Speicheranordnung 7 zugeordnet sein kann.

Die Medikamentenausgabeeinheit 3 weist ein Lagersystem 9 mit mehreren Dosen mindestens eines Medikaments auf. Hier und vorzugsweise weist das Lagersystem 9 mehrere Fächer 10 auf, wobei in jedem Fach 10 genau eine Dosis des Medikaments angeordnet sein kann. Der Begriff "Dosen" meint die Mehrzahl von Dosis, nicht von Dose.

Um die Dosen in dem Lagersystem 9 unterzubringen ist hier und vorzugsweise ein Blister vorgesehen, der mehrere, insbesondere voneinander abgegrenzte und separat zu öffnende, Fächer 10 aufweist. Der Blister weist vorzugsweise einen Identifikator, insbesondere einen RFID-Chip auf, mit dem die Unterstützungsanordnung 1, insbesondere die Medikamentenausgabeeinheit 3, prüft, welchem Patienten P der Blister zugeordnet ist. Da die Medikamentenausgabeeinheit 3 vorzugsweise einem festen Patienten P zugeordnet ist, kann die Unterstützungsanordnung 1, insbesondere die Medikamentenausgabeeinheit 3, prüfen, ob der Blister und die Medikamentenausgabeeinheit 3 dem gleichen Patienten P zugeordnet sind.

In der Speicheranordnung 7 ist ein Therapieplan mit Zeitpunkten 11 für die Einnahme der Dosen des Medikaments durch einen Patienten P gespeichert. Ein solcher Therapieplan kann beispielsweise Zeitpunkte 11 wie "morgens", "mittags", "abends" und zugeordnete Dosen umfassen. Das Lagersystem 9 kann beispielsweise 21 Fächer 10 aufweisen, um für eine Woche je drei Dosen von einem oder mehreren Medikamenten in je einem Fach 10 pro Zeitpunkt 11 zu lagern.

Die Steuereinheit 8 steuert die Medikamentenausgabeeinheit 3, um die Dosen nach dem Therapieplan zu den Zeitpunkten 11 an den Patienten P auszugeben. Zum jeweiligen Zeitpunkt 11 kann die Steuereinheit 8 ein dem Zeitpunkt 11 zugeordnetes Fach 10 öffnen und vorzugsweise den Patienten P alarmieren.

Es kann vorgesehen sein, dass die Unterstützungsanordnung 1, insbesondere die Medikamentenausgabeeinheit 3, vor der Abgabe einer Dosis an den Patienten P prüft, ob der Patient P zur Entnahme der Dosis berechtigt ist, insbesondere, ob der Patient P der Patient P ist, zu dem die Medikamentenausgabeeinheit 3 zugeordnet ist. Für diese Prüfung kann die Unterstützungsanordnung 1, insbesondere die Medikamentenausgabeeinheit 3, ein biometrisches Verfahren verwenden.

Die Medikamentenausgabeeinheit 3 kann einen Bildschirm aufweisen. Über den Bildschirm können diverse Funktionen der Medikamentenausgabeeinheit 3 wie eine Anzeige des Therapieplans, aber auch allgemeine Funktionen wie eine Videotelefonie umgesetzt werden.

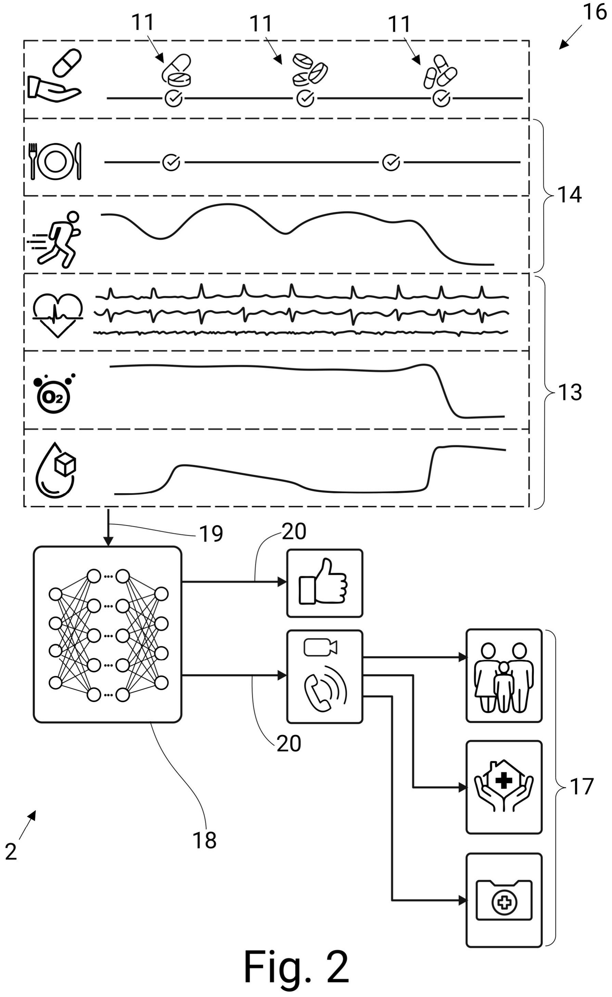

Weiter weist die Sensoranordnung 4 mindestens einen Sensor 12 auf, der Gesundheitsdaten 13 des Patienten P in Zeitspannen nach den Zeitpunkten 11 erfasst. Fig. 2 zeigt von oben nach unten jeweils auf einer gemeinsamen (schematischen) Zeitachse die Zeitpunkte 11, hier der Einnahme unterschiedlicher Kombinationen von Medikamenten, Aktivitätsdaten 14 (Mahlzeiten und Sport) und Gesundheitsdaten 13 (Puls/EKG-Daten, Sauerstoffsättigung und Blutzucker). Die Daten sind nur beispielhaft zu verstehen. Genauso ist denkbar, dass nur sporadische Pulsdaten von einer Smartwatch oder einer Ballistokardiographie-Messung zum Beispiel durch eine Kamera 15 als Sensor 12 oder von einem UWB-Sensor, Infrarot-Sensor oder dergleichen als Gesundheitsdaten 13 vorhanden sind. Beispielsweise kann der Sensor 12 für die Gesundheitsdaten 13 Teil eines Systems eines Pflegeheims sein.

Die Zeitspannen nach den Zeitpunkten 11 sind jene Zeitspannen, in denen das Medikament wirkt. Innerhalb dieser Zeitspannen müssen die Gesundheitsdaten 13 nicht durchgehend erfasst werden.

Wesentlich ist nun, dass mit dem Medikament assoziierte Wirkungsdaten über die Wirkung des Medikaments in den Zeitspannen in der Speicheranordnung 7 gespeichert sind, dass die Datenverarbeitungsanordnung 2 regelmäßig Eingangsdaten 16 miteinander korreliert und basierend auf der Korrelation einen jeweiligen Patientenzustand in eine aus mehreren Klassen einordnet, und, dass die Eingangsdaten 16 die Gesundheitsdaten 13 und die Wirkungsdaten umfassen. Der Begriff "korrelieren" meint ein miteinander in Verbindung bringen, sodass die Eingangsdaten 16, hier die Wirkungsdaten und die Gesundheitsdaten 13, zusammen einen Einfluss auf die Einordnung haben, der über eine reine Aggregation der jeweiligen Eingangsdaten 16 hinaus geht.

Die Einnahme des Medikaments etwa zum Zeitpunkt 11 wird dabei unterstellt, aber nicht zwingend kontrolliert. Wirkungsdaten von Medikamenten können ganz unterschiedliche Formen einnehmen. Für ein gegebenes Medikament kann bekannt sein, dass dieses nach 30 Min. die Pulsfrequenz um 15 % erhöht und diese Wirkung etwa eine Stunde lang anhält, bevor der Effekt zurückgeht. Diese Wirkung kann dann in den Gesundheitsdaten 13, die vorzugsweise eine Pulsfrequenz beinhalten, beobachtet werden und bei der Einordnung des Patientenzustandes berücksichtigt werden.

Die Klassen können eine Klasse aufweisen, die einem regulären Patientenzustand zugeordnet ist und eine Klasse, die einem irregulären Patientenzustand zugeordnet ist. Vorzugsweise werden mehrere Gesundheitsdaten 13 zur Einordnung in die Klassen berücksichtigt, am Beispiel der Pulsfrequenz lässt sich die vorschlagsgemäße Lehre jedoch gut erläutern. Ein Pulsfrequenz-Grenzwert für die Einordnung des Patientenzustandes in eine einem irregulären Patientenzustand zugeordnete Klasse könnte ab 30 Min. bis 90 Min. nach dem Zeitpunkt 11 der Einnahme des Medikaments um 15 % erhöht werden, um die Wirkung des Medikaments zu berücksichtigen.

Hier und vorzugsweise reagiert das Datenverarbeitungssystem auf die Einordnung des Patientenzustandes in zumindest eine der Klassen, um den Patienten P zu unterstützen. Beispielsweise kann eine der Klassen einem Herzinfarkt zugeordnet sein, worauf das Datenverarbeitungssystem einen Notruf absetzen würde.

Insbesondere zur Verbesserung der Erkennung kritischer Patientenzustände kann vorgesehen sein, dass die Klassen eine einem regulären Patientenzustand zugeordnete Klasse und mehreren jeweils einem irregulären Patientenzustand zugeordneten Klassen aufweisen. Beispielsweise kann auch ein Stresszustand als irregulärer Patientenzustand eingeordnet werden, der jedoch möglicherweise keine oder eine gemäßigtere Reaktion als einen Notruf erfordert. Durch eine detailliertere Unterteilung der Klassen können die Einordnung und die Reaktionen präziser werden. Die Klassen können auch mindestens drei oder mindestens vier einem irregulären Patientenzustand zugeordneten Klassen aufweisen.

Die einem irregulären Patientenzustand zugeordneten Klassen weisen vorzugsweise mindestens eine Klasse, insbesondere mehrere Klassen, mit einer zugeordneten Notreaktion 17 und vorzugsweise mindestens eine Klasse, insbesondere mehrere Klassen, ohne eine zugeordnete Notreaktion 17 auf. Bei Klassifikation des Patientenzustandes in die Klasse oder eine der Klassen mit einer zugeordneten Notreaktion 17 führt die Datenverarbeitungsanordnung 2 die Notreaktion 17 durch. Fig. 2 zeigt beispielhafte Notreaktionen 17, die lediglich schematisch zu verstehen sind und alternativ dazu einen Fall, in dem keine Reaktion notwendig ist.

Entsprechend sind vorzugsweise mehrere unterschiedliche Notreaktionen 17 vorgesehen. Bevorzugt ist es dabei so, dass die Notreaktionen 17 mehreren Eskalationsstufen zugeordnet sind. Die Eskalationsstufen können zum Beispiel die Eskalationsstufen "Alarmierung des Patienten" und/oder "Alarmierung eines Angehörigen" und/oder "Alarmierung eines Pflegedienstes" und/oder "Alarmierung eines Notdienstes" aufweisen. Die Medikamentenausgabeeinheit 3 kann ein, insbesondere akustisches und/oder visuelles, Ausgabegerät aufweisen. Das Ausgabegerät kann zur Alarmierung des Patienten P und/oder zur Erinnerung des Patienten P an die Medikamenteneinnahme zu den Zeitpunkten 11 genutzt werden. Der Bildschirm kann dem Ausgabegerät zugeordnet sein. Über den Bildschirm kann auch eine Kommunikation mit einem Angehörigen oder Pflegedienst im Rahmen der Alarmierung umgesetzt werden.

Die Unterstützungsanordnung 1, insbesondere die Medikamentenausgabeeinheit 3, kann weiterhin eine Flüssigkeitsaufnahme des Patienten P überwachen. Dafür kann die Medikamentenausgabeeinheit 3 einen Sensor zur Detektion einer Flüssigkeitsmenge, insbesondere eine Waage, aufweisen. Manche Patienten P, beispielsweise dialysepflichtige Patienten P, sollen ihre Flüssigkeitsaufnahme beschränken. Andere Patienten P, insbesondere ältere Patienten P, vergessen manchmal, ausreichend Flüssigkeit aufzunehmen. In beiden Fällen kann ein automatisiertes Tracking sinnvoll sein. Denkbar ist, dass Flüssigkeiten nur durch die Medikamentenausgabeeinheit 3 detektiert werden, beispielsweise indem ein Trinkgefäß auf die Waage gestellt wird, oder, dass die Unterstützungsanordnung 1 auch solche Flüssigkeiten detektieren kann, die nicht über die Medikamentenausgabeeinheit 3 detektiert werden, beispielsweise durch Bilderkennung oder eine Datenverbindung zu einem Haushaltsgerät, beispielsweise zu einer digitalisierten Kaffeemaschine. Die Unterstützungsanordnung 1 kann auch eine Dosiereinheit zur Dosierung einer Flüssigkeit aufweisen.

Detektiert die Unterstützungsanordnung 1, dass die Flüssigkeitsaufnahme des Patienten P außerhalb eines Normbereichs, insbesondere über und/oder unter einem Grenzwert, liegt, kann eine Reaktionsmaßnahme, beispielsweise eine Alarmierung des Patienten P, durchgeführt werden.

Zusätzlich kann die Unterstützungsanordnung 1, insbesondere die Medikamentenausgabeeinheit 3, einen Sensor zur Messung einer Impedanz des Pateinten über die Haut aufweisen. Von einem gemessenen Impedanzwert kann die Unterstützungsanordnung 1 auf einen Flüssigkeitshaushalt des Patienten P schließen. Die Unterstützungsanordnung 1 kann somit vorzugsweise auf die Flüssigkeitsaufnahme schließen, auch, wenn keine Daten zur Flüssigkeitsaufnahme vorliegen. Denkbar ist, dass die Unterstützungsanordnung 1 aus gemessenen Impedanzwerten und detektierten Werten zur Flüssigkeitsaufnahme mit der Zeit einen Zusammenhang zwischen Impedanzwerten und Flüssigkeitshaushalt lernt.

Es kann vorgesehen sein, dass die Datenverarbeitungsanordnung 2 zur Einordnung des Patientenzustandes in die Klassen ein trainiertes Maschinenlernmodell 18 verwendet, das durch eine Korrelation von ML-Eingangsdaten 19 ML-Ausgangsdaten 20 erzeugt. Die ML-Eingangsdaten 19 können die Eingangsdaten 16 sein oder aus den Eingangsdaten 16 abgeleitet sein. Die ML-Ausgangsdaten 20 können die Einordnung in eine Klasse umfassen oder sein.

Das Maschinenlernmodell kann durch eine Vielzahl an Gesundheitsdaten 13 von gesunden Personen darauf trainiert sein, verschiedene Patientenzustände zu erkennen. Bevorzugt sind diese Patientenzustände in Trainingsdaten des Maschinenlernmodells enthalten. Denkbar ist auch, mindestens eine Klasse vorzusehen, die nicht bekannten Klassen entspricht. Das Maschinenlernmodell kann darauf trainiert sein, einen oder mehrere Zustände zu erkennen und zu klassifizieren und zu erkennen, wann die ML-Eingangsdaten 19 keiner bekannten Klasse zugeordnet werden können.

Zusätzlich oder alternativ zur Verwendung des trainierten Maschinenlernmodells 18 können auch relative und/oder absolute, feste und/oder dynamische Grenzwerte und/oder Klasseneigenschaften durch die Datenverarbeitungsanordnung 2 zur Einordnung in die Klassen verwendet werden. Beispielsweise können bestimmte Klassen bestimmten Pulsfrequenzbereichen zugeordnet sein. Eine Pulsfrequenz von kleiner 40 oder gar 0 kann zum Beispiel nur einer einem Notfallzustand zugeordneten Klasse zugeordnet sein, sodass eine Einordnung in eine andere Klasse durch das trainierte Maschinenlernmodell 18 von vorneherein unmöglich ist.

Es kann somit vorgesehen sein, dass die möglichen Klassen, in die das trainierte Maschinenlernmodell 18 den Patientenzustand einordnen kann, abhängig von den Eingangsdaten 16 variiert werden.

Das trainierte Maschinenlernmodell 18 kann mit der Zeit lernen, welche Eingangsdaten 16 einem normalen Patientenzustand entsprechen und die zugehörige Klasse somit selbst lernen. Weiter kann das trainierte Maschinenlernmodell 18 dann erkennen, wenn eine Abweichung von dieser normalen Klasse vorliegt. Beispielsweise kann dem trainierten Maschinenlernmodell 18 ein clustering Verfahren zugrunde liegen. Die Klassen können den Clustern des clustering Verfahrens entsprechen und mit der Zeit dadurch gelernt werden, dass immer mehr Daten zur Verfügung stehen, die einem oder mehreren Normalfällen entsprechen. Liegen aktuelle Daten dann abseits aller bekannten Cluster, kann ein irregulärer Patientenzustand vorliegen.

Zur Erweiterung der Eingangsdaten 16 kann vorgesehen sein, dass die Sensoranordnung 4 mindestens einen Sensor 12 aufweist, der Aktivitätsdaten 14, insbesondere Bewegungsdaten, des Patienten P in Zeitspannen nach den Zeitpunkten 11 erfasst, und, dass die Eingangsdaten 16 die Aktivitätsdaten 14 umfassen. Aus Aktivitätsdaten 14 lassen sich diverse Rückschlüsse ziehen. Insbesondere kann der Sensor 12 ein Sensor 12 zur Detektion einer Patientenposition und/oder Bewegung, insbesondere eine Kamera 15 oder ein anderer Sensor 12 mit räumlicher Auflösung, sein. Besonders Stürze lassen sich gut aus Bewegungsdaten extrahieren. Wird beispielsweise ein Sturz mit einer Änderung der Pulsfrequenz zusammen festgestellt, kann dies ein Indiz für einen schlimmeren Sturz sein.

Die Sensoranordnung 4 kann auch mindestens einen Sensor 12, beispielsweise eine Kamera 15, aufweisen, der Stressdaten des Patienten P in Zeitspannen nach den Zeitpunkten 11 erfasst. Die Eingangsdaten 16 können dann die Stressdaten umfassen. Wird erkannt, dass ein Patient P beispielsweise gerade eine hitzige Diskussion führt, kann diese Information genutzt werden, um festzustellen, dass kein Notfall vorliegt.

Weiter ist hier und vorzugsweise vorgesehen, dass die Eingangsdaten 16 eine einem aktuellen Patientenzustand zugeordnete Klasse umfassen, sodass die Einordnung in die Klassen von der aktuellen Klasse abhängig ist. Zur Einordnung des Patientenzustandes in die Klassen kann die Datenverarbeitungsanordnung 2 einen Zustandsautomaten umsetzen. Die Übergänge zwischen den Patientenzuständen können durch das trainierte Maschinenlernmodell 18 ausgegeben werden, wobei vorzugsweise nicht aus jedem Patientenzustand ein Übergang in jeden anderen Patientenzustand möglich ist.

Zur Anpassung an den spezifischen Patienten P kann vorgesehen sein, dass die Datenverarbeitungsanordnung 2 aus den Eingangsdaten 16, insbesondere aus Eingangsdaten 16 von Zeitspannen, in denen der Patientenzustand in die dem regulären Patientenzustand zugeordnete Klasse eingeordnet ist, Referenzdaten, insbesondere Referenzgesundheitsdaten, ermittelt. Demnach kann die Datenverarbeitungsanordnung 2 eine Zeit lang mit einem Standardalgorithmus laufen und bei Einordnung des Patienten P in eine Klasse, die einem regulären Patientenzustand zugeordnet ist, Referenzdaten darüber sammeln, wie ein Normalzustand des Patienten P aussieht. Bevorzugt werden die Referenzdaten nur gesammelt, wenn die Einordnung in die Klasse, die dem regulären Patientenzustand zugeordnet ist, mit einer vorbestimmten Sicherheit erfolgt ist. So wird die Qualität der Referenzdaten sichergestellt. Vorzugsweise umfassen die Eingangsdaten 16 anschließend die Referenzdaten. Es kann auch vorgesehen sein, dass die Datenverarbeitungsanordnung 2 die Referenzdaten mit der Zeit aktualisiert.

Weiter ist hier und vorzugsweise vorgesehen, dass die Datenverarbeitungsanordnung 2 mittels der Referenzdaten die Einordnung in Klassen an den Patienten P anpasst, insbesondere individuelle Grenzwerte für die Klassen festlegt.

Denkbar ist ebenfalls, dass die Datenverarbeitungsanordnung 2 das trainierte Maschinenlernmodell 18 mit den Eingangsdaten 16 und/oder den Referenzdaten nachtrainiert.

Um die Wirkung der Medikamente zu berücksichtigen, kann vorgesehen sein, dass die Datenverarbeitungsanordnung 2 basierend auf den Wirkungsdaten die Wirkung des Medikaments in den Gesundheitsdaten 13 erkennt, und/oder, dass die Datenverarbeitungsanordnung 2 die Gesundheitsdaten 13 um einen Einfluss der Wirkung des Medikaments bereinigt. Auch können Wechselwirkungen von Medikamenten in der Speicheranordnung 7 gespeichert sein und die Datenverarbeitungsanordnung 2 kann Wechselwirkungen von Medikamenten in den Gesundheitsdaten 13 erkennen. Ist bekannt, dass ein Medikament die Pulsfrequenz um 15 % erhöht für eine gewisse Zeit, kann während dieser Zeit die Pulsfrequenz um 15 % reduziert werden (bzw. um ca. 13 % des 115 %-Wertes). So kann das trainierte Maschinenlernmodell 18 ohne Rücksicht auf die Medikamentenwirkung arbeiten, diese aber indirekt trotzdem berücksichtigen.

Möglich ist, dass die Speicheranordnung 7 lokal beim Patienten P, insbesondere an der Medikamentenausgabeeinheit 3, angeordnet ist. Das ist aus Datenschutzgründen zu bevorzugen.

Die Datenverarbeitungsanordnung 2 kann die Gesundheitsdaten 13 und die Wirkungsdaten und vorzugsweise die Stressdaten und/oder die Aktivitätsdaten 14 aggregiert und anonymisiert abspeichern. Weiter kann die Datenverarbeitungsanordnung 2 die aggregierten und anonymisierten Daten zu Studienzwecken verwenden oder zur Verfügung stellen. Das vorschlagsgemäße Verfahren ermöglicht es, eine große Menge an Daten zur Wirkung von Medikamenten zu sammeln. Diese können verwendet werden, um die Datenverarbeitungsanordnung 2 zu verbessern, aber auch, um die Medikamentenentwicklung voranzutreiben.

Weiter ist hier und vorzugsweise vorgesehen, dass die Medikamentenausgabeeinheit 3 in dem Lagersystem 9 mehrere Medikamente aufweist, die die Medikamentenausgabeeinheit 3 nach dem Therapieplan an den Patienten P ausgibt, und, dass die Wirkungsdaten Wechselwirkungsdaten der Medikamente umfassen.

Das Lagersystem 9 kann Fächer 10 zur physikalischen Freigabe der Dosen aufweist. Die Fächer 10 sind den Zeitpunkten 11 zugeordnet.

Vorgeschlagen wird gemäß einer weiteren Lehre, der eigenständige Bedeutung zukommt, eine Verwendung einer Medikamentenausgabeeinheit 3 in dem vorschlagsgemäßen Verfahren.

Auf alle Ausführungen zu dem vorschlagsgemäßen Verfahren darf verwiesen werden.

Vorgeschlagen wird gemäß einer weiteren Lehre, der eigenständige Bedeutung zukommt, ein Datenträger mit aggregierten und anonymisierten Daten erhalten durch das vorschlagsgemäße Verfahren.

Auf alle Ausführungen zu dem vorschlagsgemäßen Verfahren und der vorschlagsgemäßen Verwendung darf verwiesen werden.

Vorgeschlagen wird gemäß einer weiteren Lehre, der eigenständige Bedeutung zukommt, eine Unterstützungsanordnung 1 zur Klassifikation eines Patientenzustandes, wobei die Unterstützungsanordnung 1 eine Datenverarbeitungsanordnung 2, eine portable Medikamentenausgabeeinheit 3 und eine Sensoranordnung 4 aufweist, wobei die Datenverarbeitungsanordnung 2 eine Speicheranordnung 7 und eine Steuereinheit 8 aufweist, wobei die Steuereinheit 8 an der Medikamentenausgabeeinheit 3 angeordnet ist, wobei die Medikamentenausgabeeinheit 3 ein Lagersystem 9 mit mehreren Dosen mindestens eines Medikaments aufweist, wobei in der Speicheranordnung 7 ein Therapieplan mit Zeitpunkten 11 für die Einnahme der Dosen des Medikaments durch einen Patienten P gespeichert ist, wobei die Steuereinheit 8 die Medikamentenausgabeeinheit 3 steuert, um die Dosen nach dem Therapieplan zu den Zeitpunkten 11 an den Patienten P auszugeben, wobei die Sensoranordnung 4 mindestens einen Sensor 12 aufweist, der Gesundheitsdaten 13 des Patienten P in Zeitspannen nach den Zeitpunkten 11 erfasst.

Wesentlich nach dieser weiteren Lehre ist, dass mit dem Medikament assoziierte Wirkungsdaten über die Wirkung des Medikaments in den Zeitspannen in der Speicheranordnung 7 gespeichert sind, dass die Datenverarbeitungsanordnung 2 regelmäßig Eingangsdaten 16 miteinander korreliert und basierend auf der Korrelation einen jeweiligen Patientenzustand in eine aus mehreren Klassen einordnet, dass die Eingangsdaten 16 die Gesundheitsdaten 13 und die Wirkungsdaten umfassen.

Auf alle Ausführungen zu dem vorschlagsgemäßen Verfahren, der vorschlagsgemäßen Verwendung und dem vorschlagsgemäßen Datenträger darf verwiesen werden.

## Patentansprüche

1. Verfahren zur Klassifikation eines Patientenzustandes mittels einer Unterstützungsanordnung (1), wobei die Unterstützungsanordnung (1) eine Datenverarbeitungsanordnung (2), eine portable Medikamentenausgabeeinheit (3) und eine Sensoranordnung (4) aufweist, wobei die Datenverarbeitungsanordnung (2) eine Speicheranordnung (7) und eine Steuereinheit (8) aufweist, wobei die Steuereinheit (8) an der Medikamentenausgabeeinheit (3) angeordnet ist, wobei die Medikamentenausgabeeinheit (3) ein Lagersystem (9) mit mehreren Dosen mindestens eines Medikaments aufweist, wobei in der Speicheranordnung (7) ein Therapieplan mit Zeitpunkten (11) für die Einnahme der Dosen des Medikaments durch einen Patienten (P) gespeichert ist, wobei die Steuereinheit (8) die Medikamentenausgabeeinheit (3) steuert, um die Dosen nach dem Therapieplan zu den Zeitpunkten (11) an den Patienten (P) auszugeben, wobei die Sensoranordnung (4) mindestens einen Sensor (12) aufweist, der Gesundheitsdaten (13) des Patienten (P) in Zeitspannen nach den Zeitpunkten (11) erfasst,
**dadurch gekennzeichnet,**
**dass** mit dem Medikament assoziierte Wirkungsdaten über die Wirkung des Medikaments in den Zeitspannen in der Speicheranordnung (7) gespeichert sind, dass die Datenverarbeitungsanordnung (2) regelmäßig Eingangsdaten (16) miteinander korreliert und basierend auf der Korrelation einen jeweiligen Patientenzustand in eine aus mehreren Klassen einordnet, und, dass die Eingangsdaten (16) die Gesundheitsdaten (13) und die Wirkungsdaten umfassen.

2. Verfahren nach Anspruch 1, **dadurch gekennzeichnet, dass** die Klassen eine einem regulären Patientenzustand zugeordnete Klasse und mehreren jeweils einem irregulären Patientenzustand zugeordneten Klassen aufweisen.

3. Verfahren nach Anspruch 2, **dadurch gekennzeichnet, dass** die einem irregulären Patientenzustand zugeordneten Klassen mindestens eine Klasse, insbesondere mehrere Klassen, mit einer zugeordneten Notreaktion (17) und vorzugsweise mindestens eine Klasse, insbesondere mehrere Klassen, ohne eine zugeordnete Notreaktion (17) aufweisen, dass die Datenverarbeitungsanordnung (2) bei Klassifikation des Patientenzustandes in die Klasse oder eine der Klassen mit einer zugeordneten Notreaktion (17) die Notreaktion (17) durchführt, vorzugsweise, dass die Notreaktionen (17) mehreren Eskalationsstufen zugeordnet sind, weiter vorzugsweise, dass die Eskalationsstufen die Eskalationsstufen "Alarmierung des Patienten" und/oder "Alarmierung eines Angehörigen" und/oder "Alarmierung eines Pflegedienstes" und/oder "Alarmierung eines Notdienstes" aufweisen.

4. Verfahren nach einem der vorhergehenden Ansprüche, **dadurch gekennzeichnet, dass** die Datenverarbeitungsanordnung (2) zur Einordnung des Patientenzustandes in die Klassen ein trainiertes Maschinenlernmodell (18) verwendet, das durch eine Korrelation von ML-Eingangsdaten (19) ML-Ausgangsdaten (20) erzeugt, vorzugsweise, dass die ML-Eingangsdaten (19) die Eingangsdaten (16), und/oder, dass die ML-Ausgangsdaten (20) die Einordnung in eine Klasse umfassen oder sind.

5. Verfahren nach einem der vorhergehenden Ansprüche, **dadurch gekennzeichnet, dass** die Sensoranordnung (4) mindestens einen Sensor (12) aufweist, der Aktivitätsdaten (14), insbesondere Bewegungsdaten, des Patienten (P) in Zeitspannen nach den Zeitpunkten (11) erfasst, und, dass die Eingangsdaten (16) die Aktivitätsdaten (14) umfassen.

6. Verfahren nach einem der vorhergehenden Ansprüche, **dadurch gekennzeichnet, dass** die Sensoranordnung (4) mindestens einen Sensor (12) aufweist, der Stressdaten des Patienten (P) in Zeitspannen nach den Zeitpunkten (11) erfasst, und, dass die Eingangsdaten (16) die Stressdaten umfassen.

7. Verfahren nach einem der vorhergehenden Ansprüche, **dadurch gekennzeichnet, dass** die Eingangsdaten (16) eine einem aktuellen Patientenzustand zugeordnete Klasse umfassen, sodass die Einordnung in die Klassen von der aktuellen Klasse abhängig ist.

8. Verfahren nach einem der vorhergehenden Ansprüche, **dadurch gekennzeichnet, dass** die Datenverarbeitungsanordnung (2) aus den Eingangsdaten (16), insbesondere aus Eingangsdaten (16) von Zeitspannen, in denen der Patientenzustand in die dem regulären Patientenzustand zugeordnete Klasse eingeordnet ist, Referenzdaten, insbesondere Referenzgesundheitsdaten, ermittelt, vorzugsweise, dass die Eingangsdaten (16) anschließend die Referenzdaten umfassen, weiter vorzugsweise, dass die Datenverarbeitungsanordnung (2) die Referenzdaten mit der Zeit aktualisiert.

9. Verfahren nach Anspruch 8, **dadurch gekennzeichnet, dass** die Datenverarbeitungsanordnung (2) mittels der Referenzdaten die Einordnung in Klassen an den Patienten (P) anpasst, insbesondere individuelle Grenzwerte für die Klassen festlegt.

10. Verfahren nach einem der vorhergehenden Ansprüche, **dadurch gekennzeichnet, dass** die Datenverarbeitungsanordnung (2) basierend auf den Wirkungsdaten die Wirkung des Medikaments in den Gesundheitsdaten (13) erkennt, und/oder, dass die Datenverarbeitungsanordnung (2) die Gesundheitsdaten (13) um einen Einfluss der Wirkung des Medikaments bereinigt.

11. Verfahren nach einem der vorhergehenden Ansprüche, **dadurch gekennzeichnet, dass** die Datenverarbeitungsanordnung (2) die Gesundheitsdaten (13) und die Wirkungsdaten und vorzugsweise die Stressdaten und/oder die Aktivitätsdaten (14) aggregiert und anonymisiert abspeichert.

12. Verfahren nach einem der vorhergehenden Ansprüche, **dadurch gekennzeichnet, dass** die Medikamentenausgabeeinheit (3) in dem Lagersystem (9) mehrere Medikamente aufweist, die die Medikamentenausgabeeinheit (3) nach dem Therapieplan an den Patienten (P) ausgibt, und, dass die Wirkungsdaten Wechselwirkungsdaten der Medikamente umfassen, und/oder, dass das Lagersystem (9) Fächer (10) zur physikalischen Freigabe der Dosen aufweist, und, dass die Fächer (10) den Zeitpunkten (11) zugeordnet sind.

13. Verwendung einer Medikamentenausgabeeinheit (3) in dem Verfahren nach einem der vorhergehenden Ansprüche.

14. Datenträger mit aggregierten und anonymisierten Daten erhalten durch das Verfahren nach Anspruch 11.

15. Unterstützungsanordnung zur Klassifikation eines Patientenzustandes, wobei die Unterstützungsanordnung (1) eine Datenverarbeitungsanordnung (2), eine portable Medikamentenausgabeeinheit (3) und eine Sensoranordnung (4) aufweist, wobei die Datenverarbeitungsanordnung (2) eine Speicheranordnung (7) und eine Steuereinheit (8) aufweist, wobei die Steuereinheit (8) an der Medikamentenausgabeeinheit (3) angeordnet ist, wobei die Medikamentenausgabeeinheit (3) ein Lagersystem (9) mit mehreren Dosen mindestens eines Medikaments aufweist, wobei in der Speicheranordnung (7) ein Therapieplan mit Zeitpunkten (11) für die Einnahme der Dosen des Medikaments durch einen Patienten (P) gespeichert ist, wobei die Steuereinheit (8) die Medikamentenausgabeeinheit (3) steuert, um die Dosen nach dem Therapieplan zu den Zeitpunkten (11) an den Patienten (P) auszugeben, wobei die Sensoranordnung (4) mindestens einen Sensor (12) aufweist, der Gesundheitsdaten (13) des Patienten (P) in Zeitspannen nach den Zeitpunkten (11) erfasst,
**dadurch gekennzeichnet,**
**dass** mit dem Medikament assoziierte Wirkungsdaten über die Wirkung des Medikaments in den Zeitspannen in der Speicheranordnung (7) gespeichert sind, dass die Datenverarbeitungsanordnung (2) regelmäßig Eingangsdaten (16) miteinander korreliert und basierend auf der Korrelation einen jeweiligen Patientenzustand in eine aus mehreren Klassen einordnet, und, dass die Eingangsdaten (16) die Gesundheitsdaten (13) und die Wirkungsdaten umfassen.
